# EUROPÄISCHE PATENTSCHRIFT

(11) **EP 2 421 458 B1**
(45) Veröffentlichungstag und Bekanntmachung des Hinweises auf die Patenterteilung: **10.07.2013**
(21) Anmeldenummer: 10713587.3
(22) Anmeldetag: 14.04.2010
(51) Int. Cl.: A61B 18/02, A61B 10/02, A61B 10/04

(54) **KRYOCHIRURGISCHES INSTRUMENT**
CRYOSURGICAL INSTRUMENT
INSTRUMENT CRYOCHIRURGICAL

(30) Priorität: 21.04.2009 DE 102009018291
(43) Veröffentlichungstag der Anmeldung: 29.02.2012
(73) Patentinhaber: ERBE Elektromedizin GmbH, 72072 Tübingen (DE)
(72) Erfinder: FISCHER, Klaus, 72202 Nagold (DE); SZYRACH, Mara, 72070 Tübingen (DE); ENDERLE, Markus, 72070 Tübingen (DE)
(74) Vertreter: Rüger, Barthelt & Abel
(86) Internationale Anmeldenummer: PCT/EP2010/002291
(87) Internationale Veröffentlichungsnummer: WO 2010/121738

(56) Entgegenhaltungen:
- DE-A1-102007 020 582

## Beschreibung

Die Erfindung betrifft ein kryochirurgisches Instrument zur transbronchialen Biopsie. Bei Raumforderungen (z.B. Bronchialkarzinom oder peripherem Rundherd) bzw. entzündlichen, interstitiellen Lungenerkrankungen im peripheren Lungengewebe wird als eine Routinemethode der Diagnostik die transbronchiale Biopsie unter Durchleuchtungskontrolle angewandt. Aufgrund des Durchmessers des flexiblen Bronchoskops ist es oft unmöglich, hiermit eine Gewebeprobe aus bestimmten, weiter von den zentralen Atemwegen entfernen Bereichen der Lunge zu entnehmen. In diesem Fall wird unter Röntgenkontrolle (C-Bogen) z.B. eine feine Zange in den zu untersuchenden Bezirk vorgeschoben, um so gezielt die Gewebeproben entnehmen zu können.

Flexible Fasszangen bzw. Kryobiopsiesonden zur Durchführung dieser Untersuchung sind bekannt, etwa aus der EP 0 573 817 A1. Bei der Gewebeentnahme mit der Kryobiopsie wird die Sondenspitze, d.h. der Sondenkopf, auf das zu behandelnde Gewebe aufgesetzt und ein Gewebebereich, die Gewebeprobe, aufgrund des Kühleffekts (Joule-Thomson-Effekt) am Sondenkopf festgefroren. Das Gewebe bzw. die spätere Gewebeprobe haftet somit an dem gekühlten Sondenkopf und kann durch eine kurze Zugbewegung aus dem umliegenden Gewebe herausgelöst werden, siehe WO 2008/074422.

Bei der Gewebeentnahme im einsehbaren Bereich der Atemwege (zentrale Atemwege) erfolgt die Positionierung der Instrumente unter endoskopischer Sicht. Dagegen wird bei der Gewebeentnahme aus peripherem Lungengewebe die Positionierung der Instrumente lediglich indirekt unter Durchleuchtungskontrolle durchgeführt. Dies ist mit einer höheren Komplikationsrate verbunden. Bei der Entnahme von Gewebeproben (Biopsie) aus dem peripherem Lungengewebe ist darauf zu achten, dass das Lungengewebe, das mit einer hauchdünnen Hülle, dem Lungenfell (Pleura visceralis) überzogen ist, nicht durch z.B. zu hohe mechanische Kraft, verursacht durch das Instrument bei der Biopsie, durchstossen wird. Entsteht durch die Gewebeentnahme ein Defekt in dem Lungenfell, strömt Luft aus der Lunge in den Pleuraspalt, und es kommt somit zu einem Pneumothorax, der für den Patienten eine reduzierte Sauerstoffversorgung zur Folge hat. Wird andererseits eine Kryobiopsiesonde zu tief, also zu nahe an der Pleura positioniert, kann durch das Anfrieren des Gewebes an die Sondenspitze (Sondenkopf) auch ein Anfrieren des Lungenfells erfolgen. Bei der Extraktion des anhaftenden Gewebes an den Sondenkopf kann so ein Defekt der Pleura entstehen. Die Schwierigkeit liegt darin, dass die Instrumente bei der Gewebeentnahme aus peripherem Lungengewebe nur sehr schwer und von erfahrenen Anwendern bis kurz vor die Pleura positioniert werden können. Bei der Durchleuchtungskontrolle steht dem Anwender nur ein 2D-BiId zur Verfügung, um die Position der Sondenspitze zu kontrollieren. Es ist daher schwierig, auch die Tiefe, also die dritte Dimension, abzuschätzen.

Der Erfindung liegt die Aufgabe zugrunde, ein verbessertes Instrument der genannten Art anzugeben, das die Untersuchung für den Arzt erleichtert und das Komplikationsrisiko senkt. Diese Aufgabe wird durch ein kryochirurgisches Instrument mit den Merkmalen des Anspruchs 19 oder 10 gelöst. Zweckmäßige Fortbildungen des Erfindungsgedankens sind Gegenstand der abhängigen Ansprüche.

Ein wesentlicher Gedanke gemäß einem ersten Aspekt der Erfindung besteht darin, zusätzlich zu einem nahe dem distalen Ende angeordneten gekühlten Gewebserfassungsabschnitt, welcher derart ausgebildet ist, dass im Gebrauch des Instruments im gekühlten Zustand aufgrund von Kryoadhäsion umgebendes biologisches Material anhaftet, Mittel am Instrument vorzusehen, mit denen ein Anhaften von biologischem Material bzw. Gewebe unmittelbar am distalen Ende des Instruments verhindert wird. Dies geschieht durch einen distal vom Gewebserfassungsabschnitt vorgesehenen Sicherheitsabschnitt, welcher Mittel zum Verhindern oder Reduzieren des Anhaftens von biologischem Material aufweist. Gemäß einem zweiten, relativ unabhängigen Aspekt der Erfindung ist der Gewebserfassungsabschnitt zwar unmittelbar am distalen Ende des Instruments angeordnet, aber derart ausgebildet, dass an einer distalen Fläche, insbesondere Stirnfläche, der Kryoadhäsionseffekt gegenüber dem Umfangsbereich wesentlich reduziert ist.

In einer Ausführung gemäß dem ersten Aspekt ist der Sicherheitsabschnitt so gestaltet, dass er mindestens in einem Grenzbereich zum Gewebserfassungsabschnitt niedrige Wärmeleitfähigkeit aufweist. Hierbei kann vorgesehen sein, dass die niedrige Wärmeleitfähigkeit durch reduzierte Querschnittsfläche und/oder Auswahl eines schlecht wärmeleitenden Materials mindestens im Grenzbereich realisiert ist. Es versteht sich, dass der Sicherheitsabschnitt auch insgesamt aus schlecht wärmeleitendem Material gebildet oder mit einer gegenüber dem Gewebserfassungsabschnitt reduzierten Querschnittsfläche ausgeführt sein kann. Als Material kommen hier diverse Kunststoffmaterialien, mit ihrer typischerweise niedrigen Wärmeleitfähigkeit, in Betracht, und deren konkrete Auswahl wird unter Beachtung der speziellen Anforderungen der Medizintechnik erfolgen. Lediglich als Beispiel seien hier Silikone, Polyurethane und Polyamide genannt.

In einer weiteren Ausführung gemäß dem ersten Aspekt ist vorgesehen, dass der Sicherheitsabschnitt eine derart bemessene hohe Wärmekapazität aufweist, dass während einer kurzen Kühldauer des Gewebserfassungsabschnitts, insbesondere von weniger als 5 Sekunden, seine Temperatur oberhalb eines Werts bleibt, bei dem in Folge Kryoadhäsion biologisches Material an ihm anhaftet. Insbesondere kann er aus einem Hartmetall gebildet sein, welches typischerweise eine solche hohe Wärmekapazität aufweist. Weitere materialseitige Ausführungen erschl ießen sich dem Fachmann angesichts des konstruktiven Aufbaus des Instruments, speziell seiner Kühlung, sowie von Eigenheiten seiner Anwendung, ohne dass es dazu hier konkreterer Hinweise bedürfte.

Bei einer weiteren Ausführung gemäß dem ersten Aspekt ist der Sicherheitsabschnitt aus einem die Kryoadhäsion reduzierenden Material gefertigt oder jedenfalls mit einem solchen Material bedeckt oder mit einem Abstandhalter aus einem solchen umgeben. Insbesondere kann eine Beschichtung oder ein Abstandshalter zur Realisierung dieser Funktion aus einem die Kryoadhäsion reduzierenden Hartstoff bestehen. In einer weiteren Ausführung gemäß dem ersten Aspekt der Erfindung ist vorgesehen, dass der Sicherheitsabschnitt eine, insbesondere elektrische, Heizeinrichtung aufweist.

Mehrere der oben genannten Massnahmen können miteinander kombiniert sein. zweiten oder dritten Aspekt der Erfindung sinnvoll kombiniert sein.

Eine Ausführung gemäß dem zweiten Aspekt der Erfindung zeichnet sich durch eine derartige Ausbildung von Kühlmitteln im Gewebserfassungsabschnitt aus, dass sie nur den Umfangsabschnitt nicht aber das distale Ende kühlen, oder zwischen den Kühlmitteln und dem distalen Ende eine thermische Isolierung vorgesehen ist.

Hierdurch wird bewirkt, dass die distale Fläche benachbart zum Gewebserfassungsabschnitt eine deutlich höhere Temperatur als der Gewebserfassungsabschnitt selbst (beim Betrieb der Kühleinrichtung) behält, so dass die Temperaturerniedrigung jedenfalls nicht für einen gravierenden Kryoadhäsionseffekt ausreicht.

In einer Abwandlung dieser Ausführung ist vorgesehen, dass der Umfangsabschnitt, nicht aber das distale Ende, aus einem Material und/oder mit einer Geometrie und/oder Struktur ausgebildet ist, die das Anhaften des biologischen Materials fördern. Hier wird zwar zugelassen, dass die distale Fläche, an der aus den o.a. Gründen kein biologisches Material anhaften soll, eine im Wesentlichen ebenso niedrige Temperatur wie der Gewebserfassungsabschnitt annimmt, das Anhaften von biologischem Material an dem Letzteren wird aber durch die haftungsvermittelnde Beschichtung und/oder haftungsfördernde Geometrie bzw. Oberflächenstruktur derart gefördert, dass das (bei dieser Ausführung durchaus auftretende) Anhaften von Material an der distalen Endfläche des Instruments dem gegenüber weniger ausgeprägt ist. Es versteht sich, dass stattdessen oder in Kombination mit dieser Ausführung auch vorgesehen sein kann, die distale Endfläche mit einer Antihaftbeschichtung zu versehen oder anderweitig (z.B. durch Polieren) so auszubilden, dass die Materialanhaftung dort relativ geringer ist.

Ein ähnlicher Effekt wird bei einer weiteren Ausführung gemäß dem zweiten Aspekt der Erfindung erreicht, bei der im Gewebserfassungsabschnitt eine Vielzahl von Öffnungen vorgesehen ist, die mit einem Gaskanal im Inneren des Instrumenten-Grundkörpers in Fluidverbindung stehen. Der Gaskanal wird im Gebrauch des Instruments mit einer Ansaugeinrichtung verbunden, so dass den Gewebserfassungsabschnitt lateral umgebendes Material bzw. Gewebe an diesen angesaugt und hierdurch der Kryoadhäsionseffekt lateral verstärkt wird. Auch diese Ausführung kann mit einer gezielt haftungsvermindernden Ausgestaltung des unmittelbaren distalen Endes des Instruments kombiniert sein. Wie auch die weiter oben erwähnte Ausführung des Gewebserfassungsabschnittes mit einer Vielzahl kleiner Öffnungen (die dort eine andere Funktion erfüllt) kann die Vielzahl von Öffnungen im Gewebserfassungsabschnitt in einfacher und kostengünstiger Weise durch die Fertigung aus einem porösen Material realisiert werden.

Vorteile und Zweckmäßigkeiten der Ausführungsbeispiele ergeben sich im Übrigen aus der nachfolgenden skizzenartigen Beschreibung von Ausführungsbeispielen anhand der Figuren. Von diesen zeigen:
Fig. 1 eine schematische Darstellung einer transbronchialen Biopsie mit einem flexiblen Bronchoskop und einem eingeführten Instrument,
Fig. 2 eine schematische Darstellung einer transbronchialen Biopsie in den Bronchiolen mit einer Kryobiopsiesonde,
Fig. 3 eine schematische Darstellung einer flexiblen Kryosonde.
Fig. 4 eine schematische Darstellung einer flexiblen Kryosonde
Fig. 5 eine schematische Darstellung einer flexiblen Kryosonde,
Fig. 6 eine schematische Darstellung einer flexiblen Kryosonde,
Fig. 7 eine schematische Darstellung einer flexiblen Kryosonde,
Fig. 8 eine schematische Darstellung einer flexiblen Kryosonde,
Fig. 9 eine schematische Darstellung einer flexiblen Kryosonde, und
Fig. 10 eine schematische Darstellung einer flexiblen Kryosonde,

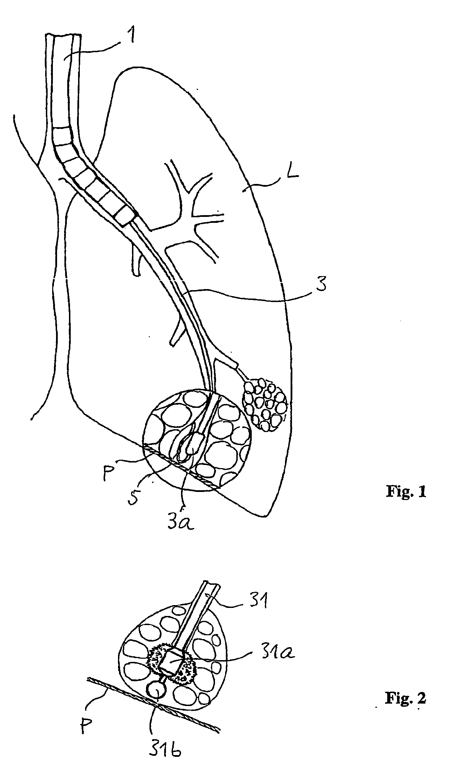

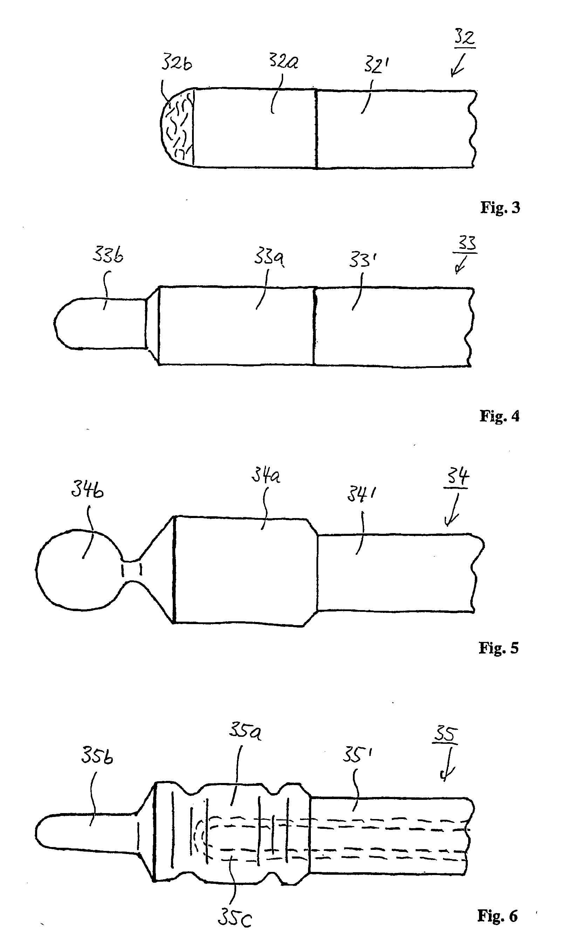

Fig. 1 zeigt in einer schematischen Längsschnittdarstellung einen Lungenflügel L mit dem distalen Ende eines eingeführten flexiblen Bronchoskops l und einer eingeführten Kryobiopsiesonde 3. Wie der vergrößerte Ausschnitt im unteren Bildteil erkennen lässt, steht aus einem distalen Sondenabschnitt 3a des Instruments 3 ein hochflexibler Führungsdraht 5 hervor, der bis an die Pleura P geführt und durch den Pleurakontakt umgebogen wurde. Die Ausführung des Führungsdrahtes 5 mit hinreichend geringer Steifigkeit bzw. hoher Biegsamkeit sowie aus einem im angewandten Durchleuchtungskontrollverfahren gut erkennbaren Material verleiht ihm die Funktion eines Markierungsmittels und erlaubt es dem handhabenden Arzt, bei Wandkontakt zur Pleura ein weiteres Vorschieben des Instruments zu unterlassen und somit eine Verletzung der Pleura zu vermeiden. Fig. 2 zeigt das distale Ende einer weiteren Kryobiopsiesonde 31, die am distalen Ende des Sondenkopfes (Gewebserfassungsabschnittes) 31a einen Abstandshalter (Sicherheitsabschnitt) 31b aufweist, der bei Inbetriebnahme einer (nicht dargestellten) Kühleinrichtung nicht gefriert und so verhindert, dass das Lungenfell P (pleura viseralis) an die Sondenspitze anfriert. Fig. 3 zeigt eine weitere Kryobiopsiesonde 32, bestehend aus einem flexiblen Schlauch 32' aus Kunststoff; mit einem Sondenkopf (Gewebserfassungsabschnitt) 32a, aus Metall, so ausgebildet, dass der Sondenkopf und der flexible Schlauch den gleichen Außendurchmesser aufweisen. Eine sphärische distale Endfläche 32b des Sondenkopfes ist hier mit einer Antihaftbeschichtung versehen, die das Anhaften von umgebendem Gewebe aufgrund des Kryoadhäsionseffektes gegenüber dem verbleibenden Umfangsbereich der Sonde 32a vermindert. Es kann eine herkömmliche und im medizinischen Einsatz bewährte Antihaftbeschichtung, etwa auf PTFE-Basis, verwendet werden.

Fig. 4 zeigt eine weitere Kryobiopsiesonde 33, bestehend aus einem flexiblen Schlauch 33', mit einer Gefriereinrichtung, einem Sondenkopf 33a und einem Abstandshalter 33b aus Kunststoff als Sondenspitze durch seine Ausführung aus Kunststoffmaterial mit niedriger Wärmeleitfähigkeit so ausgebildet, dass kein Gewebe während des Gefriervorgangs an die Sondenspitze (den Abstandshalter) anfrieren kann.

Fig. 5 zeigt eine weitere Kryobiopsiesonde 34, bestehend aus einem flexiblen Schlauch 34', mit einer (nicht dargestellten) Gefriereinrichtung im Sondenkopf 34a, die über den flexiblen Schlauch hinaus ragt, um besser Gewebe lateral anzufrieren, wobei der Sondenspitze einen Abstandshalter 34b (Sicherheitsabschnitt) aufweist, der eine schlechte Wärmeleitfähigkeit besitzt und einen schlechten Wärmekontakt (kleinen Querschnitt) zum Sondenkopf aufweist.

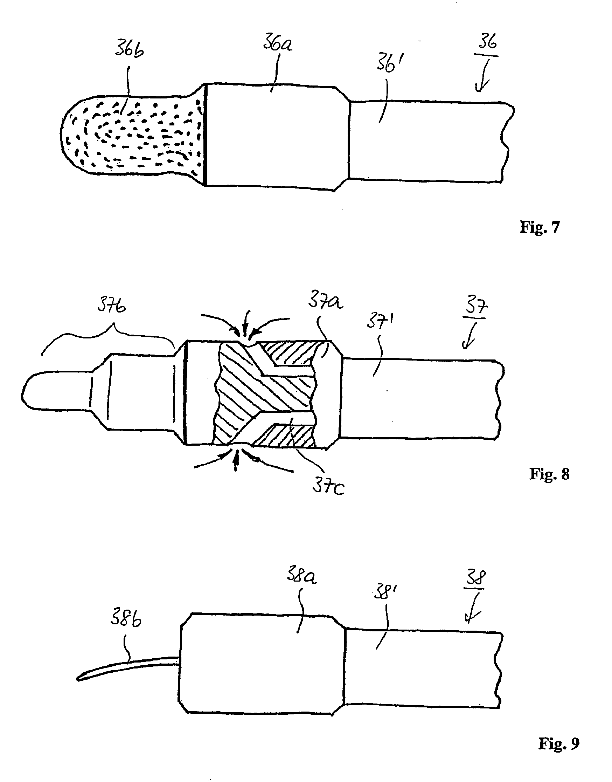

Fig. 6 zeigt eine weitere Kryobiopsiesonde 35, bestehend aus einem flexiblen Schlauch 35', mit einer Gefriereinrichtung 35c im Sondenkopf 35a, die wiederum über den flexiblen Schlauch hinaus ragt, um besser Gewebe lateral anzufrieren, wobei die Oberflächengeometrie so ausgebildet ist, dass das gefrorene Gewebe durch Formschluss (in den Vertiefungen) anhaftet, und wobei das Instrumentenende einen Abstandshalter 35b aufweist. Fig. 7 zeigt eine weitere Kryobiopsiesonde 36, bestehend aus einem flexiblen Schlauch 36', mit einer (nicht dargestellten) Gefriereinrichtung im Sondenkopf 36a und einem Abstandshalter 36b als Sondenspitze, wobei der Abstandshalter viele kleine Öffnungen aufweist, vorteilhaft aus Sinterbronze ist, wodurch während des Gefriervorgangs ein gasförmiges Medium ausströmen kann, um ein Anhaften von Gewebe zu verhindern.

Fig. 8 zeigt, teilweise im Längsschnitt, eine weitere Kryobiopsiesonde 37, bestehend aus einem flexiblen Schlauch 37' mit einer (nicht dargestellten) Gefriereinrichtung im Sondenkopf, die über den flexiblen Schlauch hinausragt, um besser Gewebe lateral anzufrieren und einem abgestuften Sicherheitsabschnitt 37b, wobei zusätzlich an dem Sondenkopf Absaugöffnungen 37c angebracht sind um das Gewebe vor dem Gefriervorgang an dem Sondenkopf mittels Vakuum (Unterdruck) zu fixieren.

Fig. 9 zeigt eine weitere Kryobiopsiesonde 38, bestehend aus einem flexiblen Schlauch 38', mit einer Gefriereinrichtung im Sondenkopf 38a und einer dünnen, hochflexiblen superelastischen Sondenspitze 38b, die sich bei geringem Widerstand umlegt oder ausweicht, und aus einem Werkstoff, der unter Durchleuchtungskontrolle sichtbar wird, siehe Fig. 1.

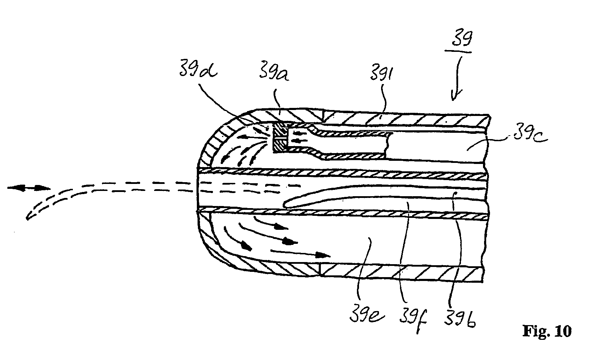

Fig. 10 zeigt in Längsschnittdarstellung das distale Ende einer weiteren Kryobiopsiesonde 39, bestehend aus einem flexiblen Schlauch 39', mit einer Gefriereinrichtung im Sondenkopf 39a, die eine oder mehrere Gaszuführung(en) 39c, eine oder mehrere Expansionsöffnung(en) 39d und eine oder mehrere Gasrückführung(en) 39e für das expandierende Gas beinhaltet, und mit einem weiteren Kanal 39f zum Einbringen eines Führungsdrahtes 39b zur Positionierung und zum Abstandkzalten zum Lungenfell (vgl. Fig. 1).

Die Ausführung der Erfindung ist nicht auf die oben skizzenhaft beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Kryochirurgisches Instrument zur transbronchialen Biopsie, mit einem langgestreckten Instrumenten-Grundkörper mit einem distalen und einem proximalen Ende, bezogen auf die Gebrauchslage, einem nahe dem distalen Ende angeordneten gekühlten Gewebserfassungsabschnitt, welcher derart ausgebildet ist, dass im Gebrauch des Instruments im gekühlten Zustand aufgrund von Kryoadhäsion umgebendes biologisches Material anhaftet **gekennzeichnet durch** einen distal vorn Gewebserfassungsabschnitt vorgesehenen Sicherheitsabschnitt, welcher Mittel zum Verhindern oder Reduzieren des Anhaftens von biologischem Material aufweist.

2. Instrument nach Anspruch 1, wobei der Sicherheitsabschnitt mindestens in einem Grenzbereich zum Gewebserfassungsabschnitt niedrige Wärmeleitfähigkeit aufweist und dazu aus einem Kunststoffmaterial besteht.

3. Instrument nach Anspruch 2, wobei die niedrige Wärmeleitfähigkeit durch reduzierte Querschnittsfläche und/oder Auswahl eines Kunststoffmaterials mindestens im Grenzbereich realisiert ist.

4. Instrument nach einem der vorangehenden Ansprüche, wobei der Sicherheitsabschnitt eine derart bemessene hohe Wärmekapazität aufweist, dass während einer kurzen Kühldauer des Gewebserfassungsabschnitts, insbesondere von weniger als 5 Sekunden, seine Temperatur oberhalb eines Werts bleibt, bei dem aufgrund von Kryoadhäsion biologisches Material an ihm anhaftet, wobei der Sicherheitsabschnitt dazu aus einem Hartmetall gebildet ist.

5. Instrument nach einem der vorangehenden Ansprüche, wobei der Sicherheitsabschnitt aus einem die Kryoadhäsion gegenüber dem Umfangsbereich reduzierenden Material besteht oder mit einem solchen bedeckt ist.

6. Instrument nach Anspruch 5, wobei der Sicherheitsabschnitt aus einem die Kryoadhäsion gegenüber dem Umfangsbereich reduzierenden Hartstoff beschichtet ist.

7. Instrument nach einem der vorangehenden Ansprüche, wobei der Sicherheitsabschnitt eine, insbesondere elektrische, Heizeinrichtung aufweist.

8. Instrument nach einem der vorangehenden Ansprüche, wobei über die Oberfläche des Sicherheitsabschnitts eine Vielzahl von Öffnungen verteilt ist, die mit einem Gaskanal im Inneren des InstrumentenGrundkörpers in Fluidveribindung stehen.

9. Kryochirurgisches Instrument zur transbronchialen Biopsie, mit einem langgestreckten Instrumenten-Grundkörper mit einem distalen und einem proximalen Ende, bezogen auf die Gebrauchslage, einem am oder nahe dem distalen Ende angeordneten gekühlten Gewebserfassungsabschnitt, welcher derart ausgebildet ist, dass im Gebrauch des Instruments im gekühlten Zustand aufgrund von Kryoadhäsion umgebendes biologisches Material anhaftet, **gekennzeichnet dadurch, dass** der Gewebserfassungsabschnitt derart ausgebildet ist, dass der Kryoadhäsionseffekt an einer distalen Fläche, insbesondere Stirnfläche, des Instruments gegenüber dem Umfang sabschnitt des Gewebserfassungsabschnitts wesentlich reduziert ist, wobei dazu Kühlmittel im Gewebserfassungsabschnitt derart ausgebildet sind, dass sie nur den Umfangsabschnitt, nicht aber die distale Fläche kühlen, oder zwischen den Kühlmitteln und der distalen Fläche eine thermische Isolierung vorgesehen ist.

10. Kryochirurgisches Instrument zur transbronchialen Biopsie, mit einem langgestreckten Instrumenten-Grundkörper mit einem distalen und einem proximalen Ende, bezogen auf die Gebrauchslage, einem am oder nahe dem distalen Ende angeordneten gekühlten Gewebserfassungsabschnitt, welcher derart ausgebildet ist, dass im Gebrauch des Instruments im gekühlten Zustand aufgrund von Kryoadhäsion umgebendes biologisches Material anhaftet, **gekennzeichnet dadurch, dass** der Gewebserfassungsabschnitt derart ausgebildet ist, dass der Kryoadhäsionseffekt an einer distalen Fläche, insbesondere Stirnfläche, des Instruments gegenüber dem Umfang sabschnitt des Gewebserfassungsabschnitts wesentlich reduziert ist, wobei der Umfangsabschnitt, nicht aber die distale Fläche mit einer Geometrie und/oder Struktur ausgebildet ist, die das Anhaften des biologischen Materials fördert,
indem im Gewebserfassungsabschnitt eine Vielzahl von Öffnungen vorgesehen ist, die mit einem Gaskanal im Inneren des Instrumenten-Grundkörpers in Fluidverbindung stehen, oder
indem der Sicherheitsabschnitt und/oder der Gewebserfassungsabschnitt aus porösem Material besteht, durch dessen Porosität die Vielzahl von Öffnungen realisiert ist.

## Claims

1. Cryosurgical instrument for transbronchial biopsy, with an elongated instrument base body with a distal and a proximal end in relation to the position of use, a cooled tissue gripping section, which is arranged close to the distal end and is configured in such a way that during use of the instrument in the cooled state surrounding biological material adheres as a result of cryoadhesion, **characterised by** a safety section that is provided distally of the tissue gripping section and has means for preventing or reducing the adhesion of biological material.

2. Instrument according to claim 1, wherein the safety section has a low thermal conductivity at least in a boundary region to the tissue gripping section and is made of a plastic material for this purpose.

3. Instrument according to claim 2, wherein the low thermal conductivity is achieved by reduced cross-sectional surface area and/or selection of a plastic material at least in the boundary region.

4. Instrument according to one of the preceding claims, wherein the safety section has a high thermal capacity dimensioned such that during a short cooling period of the tissue gripping section, in particular of less than 5 seconds, its temperature remains above a value, at which biological material adheres to it as a result of cryoadhesion, wherein the safety section is formed from a hard metal for this purpose.

5. Instrument according to one of the preceding claims, wherein the safety section is made from a material that reduces the cryoadhesion in relation to the peripheral region or is covered with such a material.

6. Instrument according to claim 5, wherein the safety section is coated with a hard material that reduces the cryoadhesion in relation to the peripheral region.

7. Instrument according to one of the preceding claims, wherein the safety section has one, in particular electrical, heating device.

8. Instrument according to one of the preceding claims, wherein a plurality of openings are distributed over the surface of the safety section that have a fluid connection with a gas duct inside the instrument base body.

9. Cryosurgical instrument for transbronchial biopsy, with an elongated instrument base body with a distal and a proximal end in relation to the position of use, a cooled tissue gripping section, which is arranged on or close to the distal end and is configured in such a way that during use of the instrument in the cooled state surrounding biological material adheres as a result of cryoadhesion, **characterised in that** the tissue gripping section is configured in such a way that the cryoadhesion effect is substantially reduced on a distal face, in particular front face, of the instrument in relation to the peripheral section of the tissue gripping section, wherein for this purpose cooling elements in the tissue gripping section are configured in such a way that they only cool the peripheral section, and not the distal face, or a thermal insulation is provided between the cooling elements and the distal face.

10. Cryosurgical instrument for transbronchial biopsy, with an elongated instrument base body with a distal and a proximal end in relation to the position of use, a cooled tissue gripping section, which is arranged on or close to the distal end and is configured in such a way that during use of the instrument in the cooled state surrounding biological material adheres as a result of cryoadhesion, **characterised in that** the tissue gripping section is configured in such a way that the cryoadhesion effect is substantially reduced on a distal face, in particular front face, of the instrument in relation to the peripheral section of the tissue gripping section, wherein the peripheral section, but not the distal face, is configured with a geometry and/or structure that promotes the adhesion of the biological material as a result of providing a plurality of openings in the tissue gripping section that have a fluid connection with a gas duct inside the instrument base body, or as a result of the safety section and/or the tissue gripping section being made from porous material, the plurality of openings being formed by the porosity thereof.

## Revendications

1. Instrument de cryochirurgie destiné à la biopsie transbronchique, comprenant un corps de base d'instrument allongé, doté d'une extrémité distale et d'une extrémité proximale, par rapport à la position d'utilisation, une partie de préhension de tissu refroidie qui est disposée à proximité de l'extrémité distale et est agencée de manière à ce que, lors de l'utilisation de l'instrument à l'état refroidi, de la matière biologique environnante adhère par cryoadhérence, **caractérisé en ce qu'**il comporte une partie de sécurité qui est prévue de façon distale par rapport à la partie de préhension de tissu et présente des moyens pour empêcher ou réduire l'adhérence de matière biologique.

2. Instrument selon la revendication 1, dans lequel la partie de sécurité présente une faible conductivité thermique, au moins dans une région limite avec la partie de préhension de tissu, et est en outre constituée d'une matière synthétique.

3. Instrument selon la revendication 2, dans lequel la faible conductivité thermique est réalisée du fait d'une aire de section transversale réduite et/ou de la sélection d'une matière synthétique au moins dans la région limite.

4. Instrument selon l'une des revendications précédentes, dans lequel la partie de sécurité présente une capacité thermique élevée qui est prévue de telle façon que, pendant une courte durée de refroidissement de la partie de préhension de tissu, notamment de moins de 5 secondes, sa température reste au-dessus d'une valeur à laquelle, du fait de la cryoadhérence, de la matière biologique adhère à cette partie, la partie de sécurité étant en outre constituée d'un métal dur.

5. Instrument selon l'une des revendications précédentes, dans lequel la partie de sécurité est constituée d'un matériau qui réduit la cryoadhérence par rapport à la région périphérique, ou est recouverte d'un tel matériau.

6. Instrument selon la revendication 5, dans lequel la partie de sécurité est recouverte d'un matériau dur réduisant la cryoadhérence par rapport à la région périphérique.

7. Instrument selon l'une des revendications précédentes, dans lequel la partie de sécurité présente un dispositif de chauffage, en particulier électrique.

8. Instrument selon l'une des revendications précédentes, dans lequel une pluralité d'orifices sont répartis sur la surface de la partie de sécurité, orifices qui sont en communication de fluide avec un conduit de gaz à l'intérieur du corps de base de l'instrument.

9. Instrument de cryochirurgie destiné à la biopsie transbronchique, comprenant un corps de base d'instrument allongé, doté d'une extrémité distale et d'une extrémité proximale, par rapport à la position d'utilisation, une partie de préhension de tissu refroidie qui est disposée sur l'extrémité distale ou à proximité de celle-ci et est agencée de manière à ce que, lors de l'utilisation de l'instrument à l'état refroidi, de la matière biologique environnante adhère par cryoadhérence, **caractérisé en ce que** la partie de préhension de tissu est agencée de manière telle que l'effet de cryoadhérence soit considérablement réduit sur une surface distale, en particulier une face frontale, de l'instrument par rapport à la région périphérique de la partie de préhension de tissu, des moyens de refroidissement étant agencés à cet effect dans la partie de préhension de tissu, de manière à ce qu'ils ne refroidissent que la région périphérique mais pas la surface distale, ou bien une isolation thermique étant prévue entre les moyens de refroidissement et la surface distale.

10. Instrument de cryochirurgie destiné à la biopsie transbronchique, comprenant un corps de base d'instrument allongé, doté d'une extrémité distale et d'une extrémité proximale, par rapport à la position d'utilisation, une partie de préhension de tissu refroidie, disposée sur l'extrémité distale ou à proximité de celle-ci, qui est agencée de manière à ce que, lors de l'utilisation de l'instrument à l'état refroidi, de la matière biologique environnante adhère par cryoadhérence, **caractérisé en ce que** la partie de préhension de tissu est agencée de manière telle que l'effet de cryoadhérence soit considérablement réduit sur une surface distale, en particulier une face frontale, de l'instrument par rapport à la région périphérique de la partie de préhension de tissu, la région périphérique, mais pas la surface distale, étant réalisée avec une géométrie et/ou une structure qui favorise l'adhérence de la matière biologique, par le fait qu'une pluralité d'orifices sont prévus dans la partie de préhension de tissu, orifices qui sont en communication de fluide avec un conduit de gaz à l'intérieur du corps de base de l'instrument, ou par le fait que la partie de sécurité et/ou la partie de préhension de tissu est/sont constituée (s) d'un matériau poreux dont la porosité crée la pluralité d'orifices.
